(19) Europäisches Patentamt
European Patent Office
Office européen des brevets

(11) Numéro de publication: **0 072 726**
**B1**

(12) # FASCICULE DE BREVET EUROPEEN

(45) Date de publication du fascicule du brevet: **20.03.85**

(51) Int. Cl.[4]: **C 07 D 237/20**

(21) Numéro de dépôt: **82401425.2**

(22) Date de dépôt: **30.07.82**

(54) **Composés aminés dérivés de la pyridazine actifs sur le système nerveux central.**

(30) Priorité: **07.08.81 FR 8115380**

(43) Date de publication de la demande:
**23.02.83 Bulletin 83/8**

(45) Mention de la délivrance du brevet:
**20.03.85 Bulletin 85/12**

(84) Etats contractants désignés:
**AT BE CH DE FR GB IT LI LU NL SE**

(56) Documents cités:
**FR - A - 2 141 697**

**CHEMICAL ABSTRACTS, vol.76, 1972, page 22, résumé no.41974h, Columbus, Ohio (US), M.R. ORNELLAS: "Biochemical studies of cerebral subfractions after chronic administration of 4-methyl-3-(2-(morpholino)ethylamino)-6-phenylpyridazine hydrochloride, AG 620"**
**CHEMICAL ABSTRACTS, vol.73, 1970, page 199, résumé no.12845z, Columbus, Ohio (US), M.R. ORNELLAS et al.: "Pharmacological interpretation of the energy metabolism of rat brain in vivo and in vitro in connection with a study on some pyridazines"**

(73) Titulaire: **SANOFI, société anonyme, 40, Avenue George V, F-75008 Paris (FR)**

(72) Inventeur: **Wermuth, Camille, 3 rue de la Côte d'Azur, F-67100 Strasbourg (FR)**
Inventeur: **Biziere, Kathleen, 6 Lotissement Rayons d'Oc, F-34170 Clapiers (FR)**
Inventeur: **Kan, Jean-Paul, 25 Lotissement l'Olivette, F-34170 Clapiers (FR)**

(74) Mandataire: **Gillard, Marie-Louise et al, Cabinet Beau de Loménie 55, Rue d'Amsterdam, F-75008 Paris (FR)**

**EP 0 072 726 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Depuis de nombreuses années, des dérivés de la pyridazine ont été proposés en tant que médicaments. Dans un grand nombre de cas, il s'agit de substances actives sur le système cardio-vasculaire et présentant en particulier un effet hypotenseur ou vasodilatateur. Plus rarement, on a mentionné parmi les dérivés de la pyridazine une action anti-inflammatoire et analgésique. Enfin le brevet FR-A N° 2141697 décrit une famille de produits répondant à la formule générale:

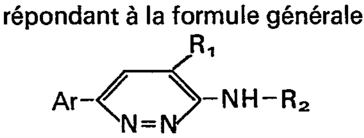

où

$R_1$ représente l'hydrogène ou un groupe alkyle inférieur

Ar représente un reste aromatique

$R_2$ désigne un groupe $-(CH_2)_n-$ 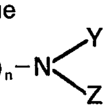 dans lequel n = 2 ou 3 et Y et Z représentent un groupe alkyle inférieur ou bien 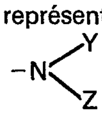 constitue un radical hétérocyclique.

Ces composés sont caractérisés par une action psychotrope de type psychostimulante.

Une étude ultérieure du composé où $R_1 = CH_3$, Ar = phényle et $R_2 = -CH_2CH_2-$ 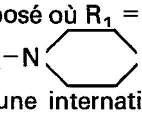 qui a reçu la dénomination commune internationale de minaprime, a permis de montrer qu'il s'agit d'une action psychotrope d'un type nouveau que l'on a désignée par activité désinhibitrice. Par ailleurs, à dose supérieure à 100 mg/kg *per os*, ce produit se montre convulsivant.

On a maintenant trouvé que certaines amino-3 pyridazines possèdent les mêmes propriétés pharmacologiques que la minaprine tout en étant moins toxiques et pratiquement dépourvues d'action convulsivante.

La présente invention a donc pour objet une famille de composés aminés dérivés de la pyridazine répondant à la formule générale (I):

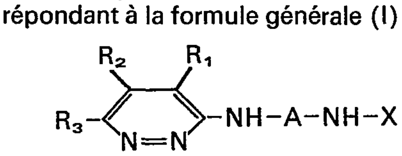 (I)

dans laquelle l'un des substituants $R_1$, $R_2$ ou $R_3$ représente un groupe phényle ou hydroxyphényle, tandis que les deux autres représentent l'hydrogène ou un groupe alkyle en $C_1$-$C_4$;

A représente un groupe alkylène droit ou ramifié ayant 2 à 5 atomes de carbone;

X représente l'hydrogène ou un groupe alkyle en $C_1$-$C_4$.

La présente invention concerne également les sels d'addition que fournissent les composés (I) avec les acides pharmaceutiquement acceptables. Elle comprend aussi un procédé de préparation des composés de formule (I) et de leurs sels ainsi que leur application en thérapeutique.

Les composés selon l'invention sont obtenus à partir d'une chloro-3 pyridazine convenablement substituée 1 par action d'une amine $H_2N-A-NH-X$ selon le schéma réactionnel:

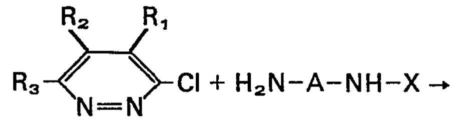 (I)

1 2

La réaction entre le dérivé chloré 1 et l'amine 2 s'effectue en général par chauffage au sein d'un solvant convenable tel qu'un alcool, le plus souvent à la température d'ébullition du solvant. La durée de la réaction varie de quelques heures à plusieurs jours suivant la nature des réactifs mis en jeu. Lorsque la réaction est trop lente, elle peut être catalysée par addition d'une petite quantité de poudre de cuivre.

On effectue la réaction en présence d'un accepteur d'hydracide destiné à fixer l'acide chlorhydrique formé; le plus souvent on utilise comme tel un excès de l'amine 2.

L'isolement du composé (I) s'effectue par reprise dans l'eau et extraction par un solvant convenable tel que l'acétate d'éthyle.

Les composés (I) peuvent être salifiés de façon habituelle par action de l'acide sur une solution chaude de la base, le solvant étant choisi de façon que le sel cristallise par refroidissement.

Les chloro-3 pyridazines utilisées comme produit de départ sont obtenues à partir des 2H-pyridazones-3 correspondantes par action d'un excès d'oxychlorure de phosphore. Les 2H-pyridazones-3, dont certaines sont connues, peuvent être obtenues par des procédés connus tels que l'action de l'hydrazine sur des acides γ-cétoniques ou des dérivés activés de ceux-ci.

Les exemples suivants sont donnés à titre d'illustration de la présente invention.

*Exemple 1:*

*(Amino-2 éthylamino)-3 méthyl-4 phényl-6 pyridazine (chlorhydrate)* (CM 300099)

(I) $R_1 = CH_3$; $R_2 = H$;

$R_3 =$ 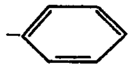

$A = (CH_2)_2$; $X = H$

On chauffe au reflux sous atmosphère inerte (argon) le mélange de 4,1 g de chloro-3 méthyl-4 phényl-6 pyridazine et 10 g de diamino-1,2 éthane.

On suit l'évolution de la réaction en prélevant un échantillon du mélange réactionnel soumis à une chromatographie en couche mince.

Lorsque le dérivé chloré a totalement disparu, on évapore l'excès d'amine à siccité. Pour éliminer les dernières traces d'amine, on reprend à deux reprises le résidu dans l'éthanol et évapore à siccité.

*Chlorhydrate:* On dissout la base hydroscopique ainsi obtenue dans le méthanol et ajoute 1,1 Eq

d'acide chlorhydrique gazeux. Par addition d'éther anhydre, le chlorhydrate précipite. Il cristallise avec 3 molécules d'eau. F: 225° C.

En opérant de la même façon, mais en remplaçant le diamino-1,2 éthane par du diamino-1,3 propane, on obtient l'(amino-3 propylamino)-3 méthyl-4 phényl-6 pyridazine (CM 30486), poudre blanche hydroscopique isolée sous forme de dichlorhydrate cristallisant avec une molécule d'eau. F: 160° C avec décomposition.

De même avec le diamino-1,4 butane, on obtient l'(amino-4 butylamino)-3 méthyl-4 phényl-6 pyridazine (CM 30487), poudre blanche hydroscopique isolée sous forme de dichlorhydrate cristallisant avec une molécule d'eau. F: 150° C avec décomposition.

*Exemple 2:*

*(Méthylamino-2 éthylamino)-2 méthyl-4 phényl-6 pyridazine* (SR 95029)

On opère comme dans l'exemple 1 en remplaçant le diamino-1,2 éthane par une quantité équivalente d'amino-1 méthylamino-2 éthane.

Par le même traitement, on obtient le produit attendu isolé sous forme de dichlorhydrate. F: 252° C.

*Exemples 3 à 6:*

En opérant comme dans l'exemple 1, mais en faisant varier d'une part les substituants des chloro-3 pyridazines 1 et/ou d'autre part les diamines 2 utilisées comme produits de départ, on obtient les divers composés (I) rassemblés dans le tableau I.

*Tableau I*

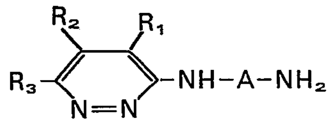

| N° de code | $R_1$ | $R_2$ | $R_3$ | A | Sel (p.f. °C) |
|---|---|---|---|---|---|
| SR 95002 | 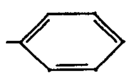 | H | H | $-(CH_2)_2-$ | Dichlorhydrate (1 mol d'eau). F.: 191 |
| SR 95030 | $-CH_3$ | H | 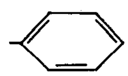 | $-CH_2-CH-CH-CH_2-$<br>\| \|<br>$CH_3$ $CH_3$ | Mélange des 2 isomères. Dichlorhydrate (½ mol d'eau). F.: 271 |
| SR 95085 | H | H | 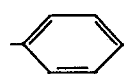 | $-(CH_2)_2-$ | Dichlorhydrate. F.: 170 |
| SR 95086 | H | 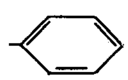 | H | $-(CH_2)_2-$ | Dichlorhydrate. F.: 200 |

*Exemple 7:*

*(Amino-2 éthylamino)-3 méthyl-4 (hydroxy-4 phényl)-6 pyridazine (dibromhydrate)* (SR 95073)

(I) $R_1 = CH_3$; $R_2 = H$;

$R_3 =$ 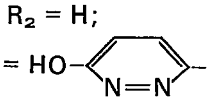 ;

$A = (CH_2)_2$; $X = H$

a) *(Amino-2 éthylamino)-2 méthyl-4 (méthoxy-4 phényl)-6 pyridazine*

On opère comme dans l'exemple 1 en remplaçant la chloro-3 méthyl-4 phényl-6 pyridazine par une quantité équivalente de chloro-3 méthyl-4 (méthoxy-4 phényl)-6 pyridazine. On obtient ainsi le produit attendu sous forme huileuse.

b) *SR 95073*

On chauffe au reflux pendant 6 h la solution de 15 g du produit obtenu ci-dessus dans 150 ml d'un mélange 2:1 (volume/volume) d'acide bromhydrique à 48% et d'acide acétique.

On évapore à siccité. Il reste une huile brunâtre qui cristallise dans le mélange éthanol/éther.

Après recristallisation dans l'éthanol, aqueux, le dibromhydrate fond à 284° C.

Les produits selon l'invention ont été soumis à des essais pharmacologiques en vue de déterminer leur activité sur le système nerveux central ainsi que leur toxicité.

*Toxicité aiguë*

Les produits à étudier ont été administrés par voie intrapéritonéale à doses croissantes à des lots de 10 souris. La mortalité provoquée par les produits étudiés a été notée pendant les 24 h ayant suivi l'administration du produit.

A partir des résultats obtenus, on détermine pour chacun des produits étudiés la dose létale 50, c'est-à-dire la dose provoquant la mort de 50% des animaux étudiés.

Pendant les mêmes expériences, on note également la dose seuil convulsivante du produit, c'est-à-dire la dose minimale pour laquelle commence à se manifester une activité convulsivante.

Les résultats obtenus sont réunis dans le tableau II. Dans ce tableau on a fait figurer, à titre de comparaison, 3 produits de l'art antérieur (FR-A N° 2141697):

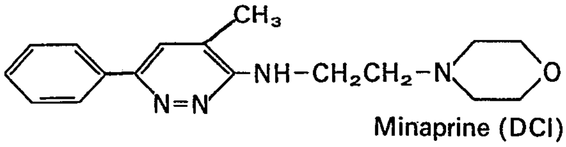

Minaprine (DCI)

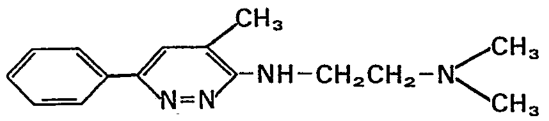

CM 30071

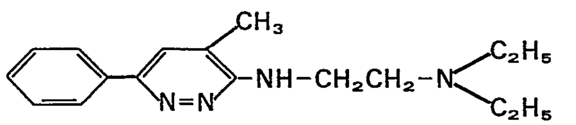

CM 30070

Les chiffres figurant dans le tableau II montrent que les produits selon l'invention présentent une toxicité et une action convulsivante très inférieures à celles des produits de référence.

*Tableau II*

*Toxicité*

| Composés | $DL_{50}$ (mg/kg; i.p.) | Dose seuil convulsivante (mg/kg; i.p.) |
|---|---|---|
| Minaprine | 63 (52-77) | 35 |
| CM 30070 | 80 (61-106) | 60 |
| CM 30071 | 118 (101-139) | 100 |
| CM 30099 | >200 | 200 |
| CM 30486 | >200 | >200 |
| CM 30487 | >200 | >200 |
| SR 95002 | ≃150 | 100 |
| SR 95029 | >200 | 200 |
| SR 95030 | >200 | >200 |
| SR 95073 | >200 | >200 |
| SR 95085 | >200 | >200 |
| SR 95086 | >200 | >200 |

*Activité antidépressive*

*Réaction de désespoir*

Ce test a été réalisé chez la souris femelle, CDI (Charles River), pesant 18 à 23 g, selon la méthode décrite par Porsolt («Archives internationales de Pharmacodynamie», 1977, *229*, 327-336).

Le principe de ce test est le suivant: lorsqu'une souris est placée dans un récipient étroit, rempli d'eau, elle se débat puis, au bout de 2 à 4 min, elle s'immobilise et flotte sur le ventre, le dos arrondi, les pattes postérieures ramenées sous le corps et elle ne fait que quelques mouvements nécessaires pour se maintenir la tête hors de l'eau. C'est la réaction dite de désespoir (despair reaction).

Certains psychotropes, notamment les antidépresseurs, allongent le temps pendant lequel la souris se débat.

Le protocole suivant a été choisi: les produits à étudier ont été administrés i.p. 1 h avant le test. Pour le test, les animaux sont placés dans un récipient étroit (10×10×10 cm), rempli d'eau, sur une hauteur de 6 cm, la température de l'eau étant de 24 ± 2° C. Les animaux sont laissés 6 min dans l'eau et on mesure le temps où l'animal reste immobile entre la 2e et la 6e min. Plus ce temps est court, plus la substance est active.

Chaque substance a été étudiée sur un lot de 10 souris. Les résultats sont la moyenne d'au moins deux expériences.

*Antagonisme de la ptôse induite par la réserpine*

Ce test, décrit par Gouret [«Journal de Pharmacologie (Paris)», 1973, *4* (1), 105-128], a été réalisé chez la souris femelle CDI (Charles River), pesant 20 ± 1 g. La réserpine entraîne une ptôsis 1 h après son administration intraveineuse; certains antidépresseurs s'opposent à cette ptôsis.

Le protocole suivant a été choisi: les substances à étudier ont été administrées i.p. La réserpine est administrée simultanément par voie intraveineuse à la dose de 2 mg/kg. 1 h après l'administration de réserpine, on note le nombre d'animaux ne présentant pas de ptôsis.

Ce test a été réalisé sur des lots de 10 souris, les résultats sont exprimés en pourcentage d'animaux ne présentant pas de ptôsis et sont la moyenne d'au moins deux expériences.

Les résultats obtenus avec les produits de l'invention figurent dans le tableau III. A titre de comparaison, on a également indiqué les résultats obtenus avec les trois produits de l'art antérieur: minaprine, CM 30070 et CM 30071.

*Tableau III*

*Activité antidépressive*

| Composés | Activité antidépressive | |
|---|---|---|
| | Antagonisme de la ptôse à la réserpine (mg/kg) | Behavioral Despair (comportement au désespoir) |
| Minaprine | $DE_{50}$ = 5 (4-7) | 10 mg/kg: −35%** |
| CM 30070 | $DE_{50} \simeq 50$ | 25 mg/kg: inactif |
| CM 30071 | $DE_{50} \simeq 50$ | 10 mg/kg: inactif |
| CM 30099 | $DE_{50}$ = 16 (14-18) | 10 mg/kg: −50%** |
| CM 30486 | $DE_{50} \simeq 10$ | 10 mg/kg: −20%* |

Des tableaux I, II et III, il ressort que les composés représentatifs de la présente invention ont, sur le plan global, une activité antidépressive du même ordre de grandeur que celle de la minaprine.

Par rapport à la minaprine et surtout aux CM 30070 et CM 30071, les produits représentatifs de la présente invention sont très peu toxiques et pratiquement dépourvus d'activité convulsivante.

Ainsi, les nouveaux composés de la présente invention peuvent être utilisés en thérapeutique dans tous les troubles du comportement psychomoteur.

Ils peuvent être prescrits, entre autres, dans la dépression masquée de l'adulte, dans les états dépressifs sérieux, dans la dépression du vieillard ainsi que dans les troubles de la mémoire et de la sénescence. Ces produits peuvent être administrés par voie orale ou par voie injectable. Les compositions pharmaceutiques peuvent être solides ou liquides et se présenter sous forme de comprimés, gélules, granulés, suppositoires ou préparations injectables.

La posologie peut varier dans de larges proportions, en particulier suivant le type et la gravité de l'affection à traiter et suivant le mode d'administration. Chez l'adulte, par voie orale, elle est le plus souvent comprise entre 0,010 et 0,500 g éventuellement répartie en plusieurs prises.

A titre d'exemple, on peut indiquer la préparation galénique suivante:

*Gélules*

| | |
|---|---|
| CM 30099 | 100 mg |
| Aérosil | 0,5 mg |
| Stéarate de magnésium | 1,5 mg |
| Amidon STA RX 1500 | 48 mg |
| | 150 mg |

**Revendications** pour les Etats contractants BE, CH, DE, FR, GB, IT, LI, LU, NL, SE

1. Dérivés aminés de la pyridazine de formule générale (I):

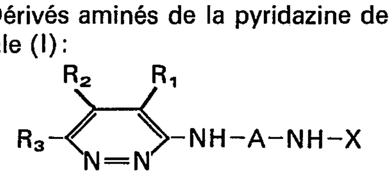
(I)

dans laquelle l'un des substituants $R_1$, $R_2$ ou $R_3$ représente un groupe phényle ou hydroxyphényle tandis que les deux autres représentent l'hydrogène ou un groupe alkyle en $C_1$-$C_4$;

A représente un groupe alkylène droit ou ramifié ayant 2 à 5 atomes de carbone;

X représente l'hydrogène ou un groupe en $C_1$-$C_4$,

ainsi que les sels desdits dérivés avec les acides pharmaceutiquement acceptables.

2. Dérivé aminé selon la revendication 1, caractérisé en ce qu'il a pour formule:

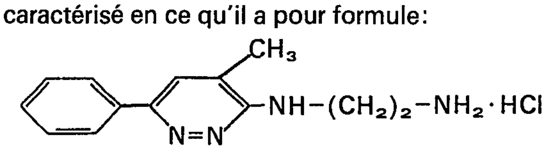

3. Procédé de préparation de dérivés aminés de formule (I) selon la revendication 1, caractérisé en ce que l'on fait réagir une chloro-3 pyridazine avec une amine de formule

$$H_2N-A-NH-X$$

la réaction s'effectuant au sein d'un solvant tel qu'un alcool à température voisine de la température d'ébullition du solvant et en présence d'un accepteur d'hydracide.

4. Médicaments utiles pour le traitement des troubles du comportement psychomoteur, caractérisés en ce qu'ils contiennent un produit, selon la revendication 1 et notamment le produit selon la revendication 2.

5. Médicaments selon la revendication 4, caractérisés en ce qu'ils contiennent de 0,01 à 0,500 g d'un produit selon la revendication 1.

**Revendications** pour l'Etat contractant: AT

1. Procédé de préparation de dérivés aminés de la pyridazine de formule (I):

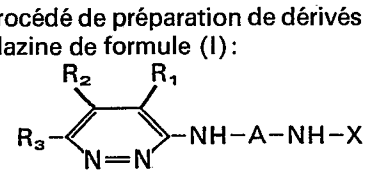
(I)

dans laquelle l'un des substituants $R_1$, $R_2$ ou $R_3$ représente un groupe phényle ou hydroxyphényle, tandis que les deux autres représentent l'hydrogène ou un groupe alkyle en $C_1$-$C_4$;

A représente un groupe alkylène droit ou ramifié ayant 2 à 5 atomes de carbone;

X représente l'hydrogène ou un groupe alkyle en $C_1$-$C_4$,

ainsi que les sels desdits dérivés avec les acides pharmaceutiquement acceptables, caractérisé en ce que l'on fait réagir une chloro-3 pyridazine avec une amine de formule

$$H_2N-A-NH-X$$

la réaction s'effectuant au sein d'un solvant tel qu'un alcool à température voisine de la température d'ébullition du solvant et en présence d'un accepteur d'hydracide.

2. Procédé selon la revendication 1, caractérisé en ce que la chloro-3 pyridazine est la chloro-3 méthyl-4 phényl-6 pyridazine et en ce que l'amine est le diamino-1,2 éthane.

**Patentansprüche** für die Vertragsstaaten BE, CH, DE, FR, GB, IT, LI, LU, NL, SE

1. Aminierte Pyridazinderivate der allgemeinen Formel (I):

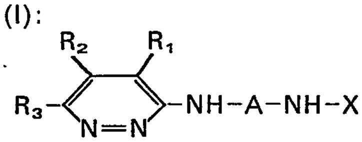
(I)

worin einer der Substituenten $R_1$, $R_2$ oder $R_3$ eine Phenyl- oder Hydroxyphenylgruppe darstellt, während die beiden anderen für Wasserstoff oder eine $C_1$- bis $C_4$-Alkylgruppe stehen,

A eine gerade oder verzweigte Alkylengruppe mit 2 bis 5 Kohlenstoffatomen bedeutet, und

X für Wasserstoff oder eine $C_1$- bis $C_4$-Alkylgruppe steht,

sowie die Salze der Derivate mit den pharmazeutisch akzeptablen Säuren.

2. Aminiertes Serivat nach Anspruch 1, dadurch gekennzeichnet, dass es folgende Formel aufweist:

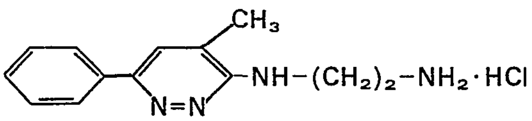

3. Verfahren zur Herstellung von aminierten Derivaten der Formel (I) nach Anspruch 1, dadurch gekennzeichnet, dass man ein 3-Chlorpyridazin mit einem Amin der Formel:

$$H_2N-A-NH-X$$

zur Umsetzung bringt, wobei die Umsetzung in einem Lösungsmittel, wie einem Alkohol, bei einer Temperatur nahe der Kochtemperatur des Lösungsmittels und in Gegenwart eines Wasserstopffsäureakzeptors erfolgt.

4. Medicamente zur Behandlung von Störungen des psychomotorischen Verhaltens, dadurdh gekennzeichnet, dass sie ein Produkt nach Anspruch 1, insbesondere das Produkt nach Anspruch 2, enthalten.

5. Medicamente nach Anspruch 4, dadurch gekennzeichnet, dass sie 0,01 bis 0,500 g eines Produkts nach Anspruch 1 enthalten.

**Patentansprüche** für den Vertragsstaat: AT

1. Verfahren zur Herstellung von aminierten Pyridazinderivaten der Formel (I):

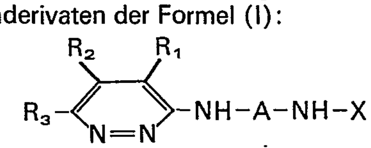 (I)

worin einer der Substituenten $R_1$, $R_2$ oder $R_3$ eine Phenyl- oder Hydroxyphenylgruppe darstellt, während die beiden anderen für Wasserstoff oder eine $C_1$- bis $C_4$-Alkylgruppe stehen,

A eine gerade oder verzweigte Alkylengruppe mit 2 bis 5 Kohlenstoffatomen bedeutet, und

X für Wasserstoff oder eine $C_1$- bis $C_4$-Alkylgruppe steht,

sowie die Salze dieser Derivate mit den pharmazeutisch akzeptablen Säuren,

dadurch gekennzeichnet, dass man ein 3-Chlorpyridazin mit einem Amin der Formel:

$$H_2N-A-NH-X$$

zur Umsetzung bringt, wobei die Umsetzung in einem Lösungsmittel, wie einem Alkohol, bei einer Temperatur nahe der Kochtemperatur des Lösungsmittels und in Gegenwart eines Wasserstoffsäureakzeptors erfolgt.

2. Verfahren nach Anspruch 1, dadurch gekennzeichnet, dass das 3-Chlorpyridazin 3-Chlor-4-methyl-6-phenylpyridazin und das Amin 1,2-Diaminoäthan ist.

**Claims** for the Contracting States BE, CH, DE, FR, GB, IT, LI, LU, NL, SE

1. Amino derivatives of pyridazine of general Formula (I):

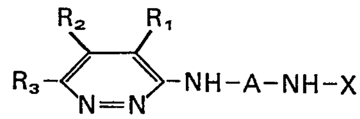 (I)

in which one of the substituents $R_1$, $R_2$ or $R_3$ represents a phenyl or hydroxyphenyl group, whilst the other two represent hydrogen or an alkyl group with $C_1$-$C_4$,

A represents a linear or branched alkylene group having 2 to 5 carbon atoms,

X represents hydrogen or an alkyl group with $C_1$-$C_4$,

as well as the salts of said derivatives with the pharmaceutically acceptable acids.

2. Amino derivative according to Claim 1, characterized in that it has the following formula:

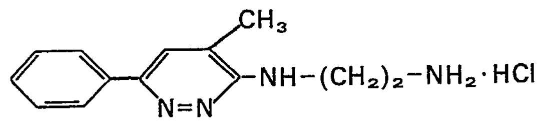

3. Process for preparing amino derivatives of Formula (I), according to Claim 1, characterized in that it comprises the step of reacting a 3-chloro pyridazine with an amine of formula:

$$H_2N-A-NH-X$$

the reaction being effected within a solvent such as an alcohol, at a temperature close to the boiling temperature of the solvent and in the presence of a hydracid acceptor.

4. Drugs useful for the treatment of disorders in the psychomotor behaviour, characterized in that they contain a product according to Claim 1, and particularly the product according to Claim 2.

5. The drugs according to Claim 4, characterized in that they contain from 0.01 to 0.500 g of a product according to Claim 1.

**Claims** for the Contracting State: AT

1. Process for the preparation of amino derivatives of pyridazine of general Formula (I):

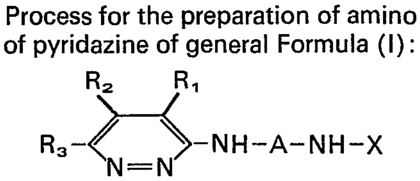 (I)

in which one of the substituents $R_1$, $R_2$ or $R_3$ represents a phenyl or hydroxyphenly group, whilst the other two represent hydrogen or an alkyl group with $C_1$-$C_4$,

A represents a linear or branched alkylene group having 2 to 5 carbon atoms,

X represents hydrogen or an alkyl group with $C_1$-$C_4$,

as well as the salts of said derivatives with the pharmaceutically acceptable acids,

characterized in that it comprises the step of reacting a 3-chloro pyridazine with an amine of formula:

$$H_2N-A-NH-X$$

the reaction being effected within a solvent such as an alcohol, at a temperature close to the boiling temperature of the solvent and in the presence of a hydracid acceptor.

2. Process according to Claim 1, characterized in that the 3-chloro pyridazine is 3-chloro 4-methyl 6-phenyl pyridazine, and in that the amine is 1,2-diamino-ethane.